Europäisches Patentamt

European Patent Office

Office européen des brevets

(19)

(11) **EP 0 795 531 B1**

(12) **FASCICULE DE BREVET EUROPEEN**

(45) Date de publication et mention
de la délivrance du brevet:
**11.07.2001 Bulletin 2001/28**

(51) Int Cl.[7]: **C07C 17/395**, C07C 21/06,
C07C 19/045, C07C 17/25,
C07C 17/02

(21) Numéro de dépôt: **97400520.9**

(22) Date de dépôt: **07.03.1997**

(54) **Procédé pour transformer les sous-produits produits de bas points d'ebbullition formes lors du craquage thermique du 1,2-dichloroéthane**

Verfahren zur Transformation der leichtsiedenden Nebenprodukte gebildet bei der thermischen Spaltung von 1,2-Dichlorethan

Process for the transformation of the by-products having a low boiling point formed during the thermal cracking of 1,2-dichloroethane

(84) Etats contractants désignés:
**AT BE CH DE DK ES FI FR GB GR IE IT LI LU NL PT SE**

(30) Priorité: **14.03.1996 FR 9603208**

(43) Date de publication de la demande:
**17.09.1997 Bulletin 1997/38**

(73) Titulaire: **Atofina
92800 Puteaux (FR)**

(72) Inventeur: **Daire, Sylvie
13220 Chateauneuf-Les-Martigues (FR)**

(56) Documents cités:
**FR-A- 2 038 347**

• **CHEMICAL ABSTRACTS, vol. 101, no. 18, 29 Octobre 1984 Columbus, Ohio, US; abstract no. 152486, BORDAS Z ET AL: "Chlorination of the thermal decomposition products of dichloroethane in producing vinyl chloride" XP002019382 & HU 30 580 O (BORSODI VEGYI KOMBINAT;HUNG.) 28 Mars 1984**

Remarques:
Le dossier contient des informations techniques présentées postérieurement au dépôt de la demande et ne figurant pas dans le présent fascicule.

**Description**

La présente invention concerne un procédé de transformation de produits secondaires formés lors du craquage thermique du 1,2-dichloroéthane lors de la préparation du chlorure de vinyle.

Les procédés industriels actuels de préparation du chlorure de vinyle sont basés sur le craquage thermique du 1,2-dichloroéthane. Généralement, le 1,2-dichloroéthane est obtenu, d'une part, par chloration en phase liquide de l'éthylène et d'autre part, par oxychloration d'éthylène en phase vapeur au moyen d'un gaz contenant de l'oxygène et de l'acide chlorhydrique lequel provient dudit craquage thermique du 1,2-dichloroéthane et/ou de toute autre source. Le craquage thermique du 1,2-dichloroéthane (désigné ci-après par DCE) est généralement effectué à des températures allant de 300° C à 650° C et sous des pressions comprises entre 8 bars et 40 bars.

Un certain nombre de sous-produits susceptibles de se trouver dans le DCE à craquer proviennent soit des produits de la dissociation thermique, recyclés, soit des produits résultant de la fabrication du DCE.

Ces sous-produits sont de nature à provoquer notamment la cokéfaction du four de craquage plus rapide que dans le cas du DCE pur. Cet encokage entraîne une élévation de la perte de charge de la zone de réaction. Il en résulte que l'on doit fréquemment arrêter la production et nettoyer cette zone. Par ailleurs, la présence de coke sur les parois du réacteur entraîne une élévation de la température dommageable pour la tenue des matériaux.

Ces sous-produits peuvent être classés en sous-produits légers et en sous-produits lourds.

On désigne présentement par sous-produits légers, ci-après désignés par légers, des produits ayant un point d'ébullition inférieur à 83,7° C et par sous-produits lourds, ci-après désignés par lourds, des produits ayant un point d'ébullition supérieur à 83,7° C.

Parmi les légers, on citera les hydrocarbures aliphatiques saturés et insaturés, éventuellement chlorés tels que le 1,3-butadiène, l'acétylène, le 2-chloro1,3-butadiène, le chloroprène, le 1,1-dichloroéthylène (trans), le 1,1-dichloroéthane, le chlorure de vinylidène, le chloroforme, le tétrachlorure de carbone, le 1,1,2-trichloroéthylène. On peut également déceler des hydrocarbures aromatiques tels que le benzène.

Certains légers sont difficiles à séparer du DCE, non transformé parce que leurs points sont très proches du point d'ébullition du DCE (PE$_{760}$ = 83.7° C) ou bien encore parce qu'ils forment avec le DCE des azéotropes. C'est le cas notamment du 1,1,2-trichloroéthylène qui est quasiment inséparable du DCE par les moyens classiques de distillation

D'une façon générale, les produits issus du craquage thermique sont séparés par des distillations successives.

Ainsi, dans une première colonne on récupère en tête l'acide chlorhydrique qui peut être utilisé pour l'oxychloration de l'éthylène. Dans une seconde colonne, on récupère en tête le chlorure de vinyle.

Dans une troisième colonne, on récupère en tête des légers, et en queue, on récupère des lourds et une quantité importante de DCE.

D'une façon générale, les lourds subissent une nouvelle distillation qui permet de récupérer le DCE non transformé, possédant une pureté suffisante pour l'introduire dans la zone de craquage thermique.

S'agissant des légers, de nombreux procédés sont connus pour les éliminer à partir du DCE non converti.

Ainsi, selon le brevet britannique GB 938824, on refroidit brutalement les produits de dissociation, constitués par du chlorure de vinyle, du gaz chlorhydrique, du DCE non converti et des sous-produits, on sépare comme mentionné précédemment par distillation, HCl, le chlorure de vinyle et ensuite on soumet les légers, nécessairement en mélange avec une partie du DCE non transformé à une élimination par distillation à la tête d'une colonne appelée colonne de fractionnement des produits à bas points d'ébullition, et on écarte le produit de distillation, tandis qu'on élimine par distillation la quantité principale du DCE non converti dans une colonne de fractionnement de produits à points d'ébullition élevés pour le séparer des produits à points d'ébullition élevés, et on le renvoie dans le serpentin de dissociation en vue d'une nouvelle dissociation. Ce procédé a comme désavantage le fait que l'on ne peut éliminer du cycle les produits à bas points d'ébullition qu'au prix de pertes élevées en DCE.

Selon un procédé décrit dans le brevet FR 2038347, on transforme les légers en produits à points d'ébullition élevés, c'est-à-dire supérieurs à 83,7 °C et on extrait le DCE pur par distillation à partir des produits à points d'ébullition élevés. Ce procédé est caractérisé par le fait que l'on charge avec du chlore gazeux le DCE impur, en présence de catalyseurs qui sont courants pour la chloration de l'éthylène en DCE, à des températures comprises entre 30° C et 85° C. De préférence, on opère en présence de 50 à 500 ppm de FeCl$_3$, par rapport au DCE, comme catalyseur. On peut procéder de manière telle que l'on envoie le DCE impur dans l'installation connue pour la réaction du chlore et de l'éthylène avec formation de DCE, on y charge conjointement l'éthylène et le DCE impur en présence du catalyseur, on sépare le catalyseur par lavage avec de l'eau et on élimine par distillation le dichloréthane pur à partir des impuretés à points d'ébullition élevés.

Selon une seconde variante, on peut procéder de manière à libérer le DCE impur, par distillation, des produits à bas points d'ébullition, retirer en continu la quantité totale ou une partie du concentrat de produits à bas points d'ébullition s'accumulant à la tête de la colonne de fractionnement des produits à bas points d'ébullition considérée, la charger en chlore gazeux en présence du catalyseur et renvoyer le mélange renfermant le catalyseur dans la colonne de fractionnement des produits à bas points d'ébullition ; à transférer le produit de fond de la colonne de fractionnement des produits à bas points d'ébullition en continu dans une colonne de fractionnement des produits à points d'ébullition élevés, et à retirer par distillation le DCE pur à partir des produits à points d'ébullition élevés renfermant le catalyseur.

Dans une troisième variante, on peut opérer de façon à libérer le DCE impur, par distillation, des produits à bas points d'ébullition, retirer en continu la quantité totale ou une partie du concentrât du produit à bas points d'ébullition accumulé à la tête de la colonne de distillation, la charger en chlore gazeux en présence du catalyseur, et renvoyer le mélange renfermant le catalyseur dans la partie inférieure de la colonne, retirer en continu le DCE pur en dessous de la tête de la colonne et évacuer les produits à points d'ébullition élevés par le fond de la colonne.

Ces différentes façons d'opérer bien qu'efficaces sur l'abaissement des teneurs de certains sous-produits, présentent cependant certains inconvénients.

Le fait d'introduire les légers dans la zone de préparation du DCE par chloration directe de l'éthylène nécessite de travailler avec un large excès de chlore, ce qui entraîne des traitements ultérieurs coûteux pour la destruction du chlore non consommé.

Par ailleurs, la chloration des légers au sein du réacteur de chloration de l'éthylène entraîne une perte importante de sélectivité de la réaction

$$Cl_2 + C_2H_4 \rightarrow C_2H_4Cl_2$$

qui se caractérise par une augmentation très sensible de la formation de 1,1,2-trichloroéthane.

La chloration des légers telle que proposée dans les variantes 2 et 3, ci-dessus mentionnées nécessite un appareillage complémentaire, une mise en oeuvre délicate au niveau du bon contrôle des réactions de chloration et surtout l'introduction de catalyseur qui devra être purgé par la suite.

Dans la demande de brevet hongrois 30580 ("Chemical, abstracts, vol. 101, N° 18, 29 octobre 1984, N° 152486"), on décrit un procédé de chloration des produits de décomposition thermique du DCE résultant du craquage dudit DCE lors de la production du chlorure de vinyle ; procédé qui consiste à soumettre sous les produits de décomposition du DCE à une chloration à une température comprise entre 20°C et 90°C, sous une pression allant de 1 à 5 bars, en présence de chlore et de HCl.

On a maintenant trouvé un procédé de transformation des sous-produits légers ayant un point d'ébullition inferieur à 83,7°C formés lors du craquage thermique du 1,2-dichloroéthane (DCE), ledit procédé étant caractérisé en ce que l'on effectue la chloration desdits sous-produits légers dans un appareillage situé directement après la préparation du 1,2-dichloroéthane par chloration directe de l'éthylène, en présence desdits produits résultant de la préparation du 1,2-dichloroéthane, à une température comprise entre 20° C et 80° C et, de préférence entre 50° C et 70° C, avec du chlore moléculaire et sans HCl. Selon la présente invention, les produits résultant de la préparation du DCE comprennent en majorité du DCE, du catalyseur et du chlore.

Selon la présente invention, on effectue avantageusement la réaction de chloration des légers en continu dans une zone homogène de réaction, en aval du réacteur de chloration directe de l'éthylène, zone dans laquelle on introduit en continu les produits résultant de la chloration directe de l'éthylène, du chlore et lesdits légers à transformer.

On opère en absence de toute radiation lumineuse.

Les produits résultant de la chloration directe de l'éthylène contiennent une quantité pondérale de chlore qui peut atteindre 20 000 ppm voire plus et qui, de préférence, comprise entre 300 ppm et 5 000 ppm.

Selon la présente invention, le catalyseur présent dans les produits résultant de la préparation du DCE par chloration directe de l'éthylène peut être un acide de Lewis tel que le chlorure ferrique.

Selon la présente invention, la teneur en catalyseur dans les produits résultant de la préparation du DCE peut être comprise entre 20 ppm et 200 ppm et, de préférence, comprise entre 30 ppm et 80 ppm

On ne sortirait pas du cadre de l'invention si l'on introduisait en supplément dans la zone de transformation des légers du catalyseur tel que défini précédemment

Selon la présente invention, le chlore qui est introduit se dissout dans le milieu réactionnel et sa concentration est avantageusement maintenue entre 100 mg/kg et 2000 mg/kg de produit sortant de la zone de réaction de chloration des légers et, de préférence une concentration comprise entre 200 mg/kg et 500 mg/kg.

Selon la présente invention, le temps de séjour est au moins égal à 10 minutes et, de préférence compris entre 20 minutes et 40 minutes.

Le chlore moléculaire mis en oeuvre, selon l'invention, peut être soit sous forme liquide que l'on gazéifie avant mise en réaction, soit sous forme de chlore gazeux à l'état brut, tel qu'il est recueilli à la sortie des ateliers de fabrication du chlore par électrolyse de solutions aqueuses de chlorure de sodium. C'est ainsi qu'il est pratiquement équivalent d'employer du chlore liquide à 99,9 % de pureté ou un chlore à 95 % de pureté, les principales impuretés étant constituées par du $CO_2$, $O_2$, $N_2$, $H_2$ et CO. Ces gaz sont inertes dans les conditions opératoires de la réaction. Le chlore utilisé peut être dilué par des gaz inertes comme par exemple les gaz que l'on vient de citer. Une telle dilution dans un rapport molaire diluant/chlore pouvant atteindre 1/1 n'est pas nuisible à la réaction.

Selon la présente invention, la chloration des légers est avantageusement réalisée au sein de l'unité de chloration directe dans un appareillage existant tel que bac tampon, situé en aval dudit réacteur de chloration directe.

Les produits sortant de la zone de chloration des légers selon la présente invention subissent avantageusement deux lavages, le premier à l'eau, le second au moyen d'une solution alcaline, puis sont soumis à une distillation fractionnée permettant de séparer le DCE des légers chlorés et des impuretés issus de la réaction de chloration directe de l'éthylène. Le DCE ainsi obtenu possède une pureté (suffisante) permettant son envoi au craquage thermique.

Le procédé selon l'invention entraîne une consommation de chlore moins importante ainsi qu'une utilisation de solution alcaline moindre.

De plus, le procédé selon la présente invention permet de conserver une bonne sélectivité à la réaction de chloration directe de l'éthylène.

Notons également que la réaction de chloration des légers selon la présente invention ne nécessite pas de matériel complémentaire puisqu'elle s'effectue au sein de l'unité de chloration directe de l'éthylène.

L'exemple qui suit illustre l'invention.

**EXEMPLE**

On effectue la réaction de chloration de l'éthylène dans un réacteur en acier ordinaire équipé d'un système de thermorégulation pour maintenir constante la température à l'intérieur du réacteur.

Le flux de produits qui sort de ce réacteur présente les caractéristiques suivantes :

- débit :        38,5 t/h
- température :        62° C
- pression atmosphérique
- chlore :        1000 ppm
- chlorure ferrique :        30 ppm (exprimé en fer)
- pureté du 1,2-dichloroéthane :        99,8 % (en poids)

Ce flux de produit s'écoule dans un bac tampon récupérant également le DCE provenant de la condensation des évents provenant de la chloration directe.

On introduit au même niveau que ce flux les légers issus de la section du craquage thermique de DCE et séparés au niveau de la colonne de fractionnement des produits à points d'ébullition élevés.

Ce flux de légers a les caractéristiques suivantes :

- débit : 2300 kg/h
- composition pondérale :

    DCE : 90 %
    légers: 10 %

Les légers sont constitués notamment par : du benzène, du chlorure de vinyle, du chlorure de vinylidène, du trans dichloroéthylène, du chloroprène, du 2-chloro-1,3-butadiène, du 1,1,2-trichloroéthylène, du chloroforme, du tétrachlorure de carbone.

Le temps de séjour dans le bac tampon est de 20 minutes et la température voisine de 60° C.

Un appoint de chlore est effectué afin de maintenir un excès de chlore dans le produit sortant de 300 ppm.

Les produits sortant du bac tampon sont lavés à l'eau, puis ensuite avec une solution de soude et soumis à une distillation fractionnée.

On récupère 200 kg/h d'impuretés lourdes ayant consommé au total 120 kg/h de chlore dont environ 30 kg/h provenant du flux sortant du réacteur de chloration directe et 90 kg/h du chlore d'appoint.

Le flux sortant du bac tampon ne contient plus notamment de benzène, de chlorure de vinyle, de chloroprène.

On n'observe aucune dégradation de la qualité du DCE formé dans le réacteur de chloration directe alors que la chloration des légers dans le réacteur de chloration directe de l'éthylène entraîne notamment la dégradation d'une quantité pondérale de DCE qui est égale à 6 000 ppm.

**Revendications**

1. Procédé de transformation des sous-produits légers ayant un point d'ébullition inférieur à 83,7°C formés lors du craquage thermique du 1,2-dichloroéthane (DCE), ledit procédé étant caractérisé en ce que l'on effectue la chloration desdits sous-produits légers dans un appareillage situé directement après la préparation du 1,2-dichloroéthane par chloration directe de l'éthylène, en présence desdits produits résultant de la préparation du 1,2-dichloroéthane, à une température comprise entre 20° C et 80° C, avec du chlore moléculaire et sans HCl.

2. Procédé selon la revendication 1, caractérisé en ce que les produits résultant de la préparation du 1,2-dichloroéthane par chloration directe de l'éthylène comprennent en majorité du 1,2-dichloroéthane, du catalyseur et du chlore.

3. Procédé selon la revendication 1, caractérisé en ce que la température est comprise entre 50° C et 70° C.

4. Procédé selon la revendication 1, caractérisé en ce que la concentration en chlore moléculaire est maintenue entre 100 mg/kg et 2000 mg/kg de produit sortant de la zone de réaction de chloration des légers.

**5.** Procédé selon la revendication 4, caractérisé en ce que la concentration en chlore moléculaire est comprise entre 200 mg/kg et 500 mg/kg.

**6.** Procédé selon l'une des revendications 1 à 5, caractérisé en ce que le temps de séjour est au moins égal à 10 minutes.

**7.** Procédé selon la revendication 6, caractérisé en ce que le temps de séjour est compris entre 20 minutes et 40 minutes.

**8.** Procédé selon la revendication 2, caractérisé en ce que le catalyseur dans les produits résultant de la préparation du 1,2-dichloroéthane est du chlorure ferrique

**9.** Procédé selon la revendication 2, caractérisé en ce que la teneur en catalyseur dans les produits résultant de la préparation du 1,2-dichloroéthane est comprise entre 20 ppm et 200 ppm.

**Claims**

**1.** Process for converting the light by-products having a boiling point lower than 83.7°C formed during the thermal cracking of 1,2-dichloroethane (DCE), the said process being characterized in that chlorination of the said light by-products is carried out in apparatus located directly after preparation of the 1,2-dichloroethane by direct chlorination of ethylene, in the presence of the said products resulting from the preparation of the 1,2-dichloroethane, at a temperature of between 20°C and 80°C, with molecular chlorine and without HCl.

**2.** Process according to Claim 1, characterized in that the products resulting from the preparation of the 1,2-dichloroethane by direct chlorination of ethylene mainly comprise 1,2-dichloroethane, catalyst and chlorine.

**3.** Process according to Claim 1, characterized in that the temperature is between 50°C and 70°C.

**4.** Process according to Claim 1, characterized in that the concentration of molecular chlorine is maintained between 100 mg/kg and 2000 mg/kg of product leaving the zone for the chlorination reaction of the lights.

**5.** Process according to Claim 4, characterized in that the concentration of molecular chlorine is between 200 mg/kg and 500 mg/kg.

**6.** Process according to one of Claims 1 to 5, characterized in that the residence time is at least equal to 10 minutes.

**7.** Process according to Claim 6, characterized in that the residence time is between 20 minutes and 40 minutes.

**8.** Process according to Claim 2, characterized in that the catalyst in the products resulting from the preparation of the 1,2-dichloroethane is ferric chloride.

**9.** Process according to Claim 2, characterized in that the content of catalyst in the products resulting from the preparation of the 1,2-dichloroethane is between 20 ppm and 200 ppm.

**Patentansprüche**

**1.** Verfahren zur Umwandlung von leichten Nebenprodukten mit einem Siedepunkt unter 83,7 °C, die sich beim thermischen Cracken von 1,2-Dichlorethan (DCE) bilden, wobei sich das genannte Verfahren dadurch auszeichnet, daß man die Chlorierung der genannten leichten Nebenprodukte in einer Vorrichtung durchführt, die direkt hinter der Herstellung des 1,2-Dichlorethans durch direkte Chlorierung des Ethylens angeordnet ist, in Gegenwart der genannten aus der Herstellung des 1,2-Dichlorethans hervorgehenden Produkte bei einer Temperatur zwischen 20 °C und 80 °C mit molekularem Chlor und ohne HCl.

**2.** Verfahren nach Anspruch 1, dadurch gekennzeichnet, daß die aus der Herstellung des 1,2-Dichlorethans durch direkte Chlorierung des Ethylens hervorgehenden Produkte zum größten Teil 1,2-Dichlorethan, einen Katalysator und Chlor enthalten.

**3.** Verfahren nach Anspruch 1, dadurch gekennzeichnet, daß die Temperatur zwischen 50 °C und 70 °C liegt.

**4.** Verfahren nach Anspruch 1, dadurch gekennzeichnet, daß die Konzentration von molekularem Chlor zwischen 100 mg/kg und 2.000 mg/kg des aus der Reaktionszone der Chlorierung der Leichtstoffe austretenden Produkts gehalten wird.

**5.** Verfahren nach Anspruch 4, dadurch gekennzeichnet, daß die Konzentration von molekularem Chlor zwischen 200 mg/kg und 500 mg/kg liegt.

**6.** Verfahren nach einem der Ansprüche 1 bis 5, dadurch gekennzeichnet, daß die Verweilzeit mindestens 10 Minuten beträgt.

**7.** Verfahren nach Anspruch 6, dadurch gekennzeichnet, daß die Verweilzeit zwischen 20 Minuten und 40 Minuten liegt.

8. Verfahren nach Anspruch 2, dadurch gekennzeichnet, daß der Katalysator in den aus der Herstellung des 1,2-Dichlorethans hervorgehenden Produkten Eisen-III-chlorid ist.

9. Verfahren nach Anspruch 2, dadurch gekennzeichnet, daß der Gehalt des Katalysators in den aus der Herstellung des 1,2-Dichlorethans hervorgehenden Produkten zwischen 20 ppm und 200 ppm liegt.